# EUROPÄISCHE PATENTSCHRIFT

(11) **EP 2 642 950 B1**
(45) Veröffentlichungstag und Bekanntmachung des Hinweises auf die Patenterteilung: **22.04.2015**
(21) Anmeldenummer: 11799083.8
(22) Anmeldetag: 25.11.2011
(51) Int. Cl.: A61F 2/26

(54) **IMPLANTATIONSHILFE FÜR EIN IMPLANTIERBARES RESERVOIR, IMPLANTATIONSEINHEIT UND VERFAHREN ZUR HERSTELLUNG EINER IMPLANTATIONSEINHEIT MIT IMPLANTIERBAREM RESERVOIR**
IMPLANTATION AID FOR AN IMPLANTABLE RESERVOIR, IMPLANTATION UNIT, AND METHOD FOR PRODUCING AN IMPLANTATION UNIT WITH IMPLANTABLE RESERVOIR
AIDE D'IMPLANTATION POUR UN RÉSERVOIR IMPLANTABLE, UNITÉ D'IMPLANTATION ET PROCÉDÉ POUR LA FABRICATION D'UNE UNITÉ D'IMPLANTATION COMPORTANT UN RÉSERVOIR IMPLANTABLE

(30) Priorität: 26.11.2010 DE 102010062072
(43) Veröffentlichungstag der Anmeldung: 02.10.2013
(73) Patentinhaber: ET Elastomer Technik GmbH, 74248 Ellhofen (DE)
(72) Erfinder: KÖNIG, Jochen, 74626 Bretzfeld (DE)
(74) Vertreter: Emig, Ralf
(86) Internationale Anmeldenummer: PCT/EP2011/071051
(87) Internationale Veröffentlichungsnummer: WO 2012/069643

(56) Entgegenhaltungen:
- US-A- 4 244 370
- US-A- 5 484 450
- US-A1- 2004 010 244

## Beschreibung

Die Erfindung betrifft eine Implantationshilfe für ein in einen menschlichen oder tierischen Körper implantierbares, mit Fluid befüllbares Reservoir einer Penisprothese sowie eine Implantationseinheit aufweisend eine Implantationshilfe und ein Reservoir und ein Verfahren zur Herstellung einer Implantationseinheit.

Die Implantierung üblicherweise aus einem flexiblen oder elastischen Material gefertigter Reservoirs ist beispielsweise bei Penisprothesen notwendig. Penisprothesen stellen dabei eine bekannte Methode zur Behandlung von erektiler Dysfunktion bei männlichen Patienten dar. Die Penisprothese wird hierbei dazu verwendet, eine künstliche Erektion bei dem Patienten zu erzeugen, was diesem ermöglicht, sexuell wieder aktiv zu werden.

Üblicherweise besteht eine solche Prothese aus einem oder zwei mit Fluid befüllbaren, zum Beispiel aufpump- oder aufblasbaren Zylindern, die in das Corpus cavernosum penis, also in den Penisschwellkörper, des Patienten implantiert werden. Dieser bzw. diese Zylinder sind mittels Leitungen mit einer Ventile aufweisenden Pumpeinheit fluidtechnisch verbunden, die ihrerseits mit einem Reservoir für eine Kochsalzlösung oder eine andere biokompatible Flüssigkeit gekoppelt ist. Wird die Pumpeinheit betätigt, wird in dem Reservoir enthaltenes Fluid von dem Reservoir durch die Leitungen in den oder die Zylinder gepumpt. Ein flexibler Zylinder wird somit unter Druck gesetzt und versteift sich unter diesem aufgebauten Druck. Die gewünschte Erektion wird erzeugt.

Will der Patient wieder in den nicht-erigierten Zustand wechseln, muss das Fluid von dem oder den Zylindern wieder in das Reservoir gebracht werden. Hierzu wird üblicherweise ein Ventil der Pumpeinheit betätigt und/oder die Zylinder werden komprimiert.

Eine derartige Penisprothese mit zwei Zylindern und einem Reservoir ist beispielsweise in der US-A 4,566,446 offenbart.

Alle Penisprothesen mit Fluid befüllbaren Zylindern haben ein Reservoir, in dem das Fluid gespeichert ist, wenn die Zylinder entleert sind. Dieses Reservoir wird regelmäßig im Unterbauch des Patienten platziert. Dabei wird in der Praxis die Platzierung des Reservoirs je nach Ausführung entweder vor oder hinter dem Beckenknochen des Patienten vorgenommen. Maßgeblich für die Platzierung ist hierbei die Höhe des Reservoirs. Reservoirs mit einer Höhe von mehr als 30 mm werden aus Gründen des Komforts hinter dem Beckenknochen platziert, wo sie nicht ertastet werden können und sich während des notwendigen chirurgischen Eingriffs mit den Fingern eine Kavität schaffen lässt. Reservoirs mit einer Höhe von unter 30 mm können auch vor dem Beckenknochen über den Bauchmuskeln platziert werden.

Die Platzierung eines Reservoirs hinter dem Beckenknochen erfolgt blind. Der Chirurg hat keine Möglichkeit, die Position des Reservoirs optisch zu überprüfen. Zum Platzieren des Reservoirs muss der Chirurg das Reservoir mit den Fingern in die von ihm geschaffene Kavität einführen und gegebenenfalls mit einer Klemme oder einem vergleichbaren anderen medizinischen Instrument in die endgültige Lage bringen.

Bei diesem Verfahren besteht die Gefahr, das Reservoir mit dem medizinischen Instrument zu beschädigen. Außerdem kann die Position des Reservoirs nicht überprüft bzw. die bestimmungsgemäße Position nicht ohne Weiteres sichergestellt werden, da der Chirurg keinen Sichtkontakt mit dem Reservoir hat. Oftmals wird daher ein solches Reservoir mittels eines (zusätzlichen) Einschnitts in die Bauchdecke platziert. Somit entsteht ein weiterer potentieller Infektionsherd.

Die vorgenannten Probleme treten jedoch nicht nur im Zusammenhang mit der Implantation von (Flüssigkeit-) Reservoirs für Penisprothesen, sondern auch bei der Implantation vergleichbarer Reservoirs auf, die an nur schwer zugänglichen bzw. einsehbaren Stellen innerhalb des Körpers bestimmungsgemäß zu platzieren sind. Hierbei wird unter einem Reservoir grundsätzlich jedwedes Behältnis verstanden, das zumindest teilweise (auch) mit Fluid befüllbar ist, zum Beispiel auch ein Behälter oder ein Beutel.

Aus dem Dokument US 2004/0010244 A1 ist eine gattungsgemäße Implantationshilfe für ein in einen menschlichen oder tierischen Körper implantierbares, mit Fluid befüllbares Reservoir einer Penisprothese bekannt.

Es ist Aufgabe der vorliegenden Erfindung, eine Implantationshilfe oder eine Implantationseinheit bereitzustellen, die den Implantationsvorgang insbesondere für ein implantierbares Reservoir einer Penisprothese vereinfacht.

Diese Aufgabe wird sowohl mit der Implantationshilfe nach Anspruch 1 als auch mit der Implantationseinheit nach Anspruch 11 sowie einem Verfahren zur Herstellung einer Implantationseinheit nach dem Anspruch 12 gelöst.

Gemäß dem Anspruch 1 ist dabei eine Implantationshilfe vorgesehen, die ein Lagerteil aufweist, an dem ein implantierbares, mit Fluid befüllbares Reservoir einer Penisprothese vor seiner Implantierung festlegbar ist und das einen relativ zu dem Lagerteil entlang einer Ausbringungsrichtung verstellbaren Schlitten aufweist. Mittels des Schlittens kann das zu implantierende Reservoir in die Ausbringungsrichtung verschoben werden.

Folglich ist der Schlitten der Implantationshilfe verschieblich gelagert und ist zwischen einem eingefahrenen und einem ausgefahrenen Zustand (stufenlos) verschiebbar. Ein Operateur kann somit eine Implantationshilfe mit daran festgelegtem Reservoir in den Körper des Patienten einführen und durch Verschiebung des Schlittens in Ausbringungsrichtung das zu implantierende Reservoir in einer vorgegebenen Lage bzw. Ausrichtung platzieren. Nach der Ausbringung und Platzierung des Reservoirs kann dieses von der Implantationshilfe getrennt und die Implantationshilfe entfernt werden.

Durch die Bereitstellung einer erfindungsgemäßen Implantationshilfe ist somit nicht nur sichergestellt, dass ein damit implantiertes Reservoir stets in identischer Weise durch Verstellung des Schlittens platziert werden kann, sondern es kann auch vorgesehen sein, dass die Implantationshilfe wiederholt einsetzbar ist, indem sie nach einer Implantierung eines Reservoirs wieder mit einem neuen Reservoir bestückt wird.

In einer bevorzugten Ausführungsform ist der Schlitten der Implantationshilfe derart ausgebildet, dass das zu implantierende Reservoir an dem Schlitten anbringbar ist. Demgemäß ist das an dem Schlitten angebrachte Reservoir im eingefahrenen Zustand des Schlittens an dem Lagerteil festgelegt. Durch Anbringung des zu implantierenden Reservoirs an dem Schlitten wird die Bestückung einer erfindungsgemäßen Implantationshilfe erheblich erleichtert, da der Schlitten zunächst ausgefahren werden kann und damit besser zugänglich ist. Nach der Anbringung des Reservoirs kann der Schlitten wiederum eingefahren werden, so dass das Reservoir in einer vor Beschädigungen geschützten Ausgangslage vorliegt, in der es mittels der Implantationshilfe in den Körper des Patienten einführbar ist.

Ein mit einer erfindungsgemäßen Implantationshilfe zu implantierendes Reservoir kann auf unterschiedliche Weise ausgebildet sein. So ist beispielsweise ein Reservoir denkbar, das eine sphärische Form aufweist. Gleichfalls ist ein Reservoir denkbar, das eine zylindrische, kugelförmige, scheibenartige oder quaderförmige Form aufweist.

Unabhängig von der Form sind derartige Reservoirs bevorzugt aus einem flexiblen oder sogar (teil-) elastischen Material gefertigt, um sie besser dem Körper des Patienten anpassen zu können. Es hat sich deshalb als vorteilhaft herausgestellt, an dem Schlitten wenigstens ein stabförmiges Führungsmittel vorzusehen, mittels dem das Reservoir in einer vorgegebenen Lage entlang der Ausbringungsrichtung verschiebbar ist und/oder an dem das Reservoir befestigbar ist. Über ein solches längserstrecktes, stabförmiges Führungsmittel, das folglich gleichfalls entlang der Ausbringungsrichtung verstellbar ist, kann einem zumindest abschnittsweise flexiblen oder sogar elastisch ausgebildeten Reservoir eine gewünschte Lage bzw. Ausrichtung aufgezwungen werden, die es auch während der Verschiebung über den Schlitten während des Implantationsvorgangs beibehält.

In diesem Zusammenhang wird es ferner als vorteilhaft angesehen, dass ein zumindest abschnittsweise flexibel oder elastisch ausgebildetes Reservoir in einem verformten, insbesondere gefalteten oder gewickelten Zustand und damit in einer kompakteren Form an der Implantationshilfe festlegbar ist. So ist in einer Ausführungsform vorgesehen, dass ein solches Reservoir zumindest abschnittsweise um ein stabförmiges Führungsmittel wickelbar oder faltbar ist und zur Implantierung in dem gewickelten bzw. gefalteten Zustand in Ausbringungsrichtung durch den Schlitten verschiebbar ist. Nach der bestimmungsgemäßen Positionierung über die Implantationshilfe wird dann das Reservoir zum Beispiel durch Befüllen mit Fluid oder aufgrund seiner (teil-) elastischen Ausbildung die gewünschte (ursprüngliche) Form einnehmen.

Hierbei sei erwähnt, dass unter einer flexiblen Ausgestaltung verstanden wird, dass ein Reservoir bzw. ein Abschnitt (der Hülle) des Reservoirs verformbar ist, wobei das Material nicht ohne Krafteinwirkung von außen (exakt) in die Ursprungsform zurückkehrt, sondern in einem verformten Zustand verbleibt oder sich zumindest nach Wegfall der die Verformung hervorrufenden, von außen angreifenden Kraft der ursprünglichen Form allenfalls wieder annähert. Dies ist zu unterscheiden von einer (teil-) elastischen Ausgestaltung, bei der ein Reservoir stets bestrebt ist, in eine ursprüngliche Form (Ursprungsform) zurückzukehren, sobald eine die Verformung hervorrufende Kraft wegfällt.

Ferner ist es vorzuziehen, dass ein Reservoir zunächst in einem unbefüllten oder zumindest im Wesentlichen entleerten Zustand implantiert wird, um es leichter platzieren zu können. So wird beispielsweise bei dem operativen Eingriff zur Platzierung eines Flüssigkeitsreservoirs für eine Penisprothese das Reservoir regelmäßig zunächst unbefüllt bzw. leer im Körper des Patienten positioniert und erst anschließend mit dem vorgesehenen Fluid gefüllt. Dementsprechend wird eine Ausführung einer Implantationshilfe bevorzugt, bei der das zu implantierende Reservoir in einem (im Wesentlichen) unbefüllten Zustand an der Implantationshilfe festlegbar ist, in dem das Reservoir üblicherweise weniger steif und damit schwieriger bestimmungsgemäß positionierbar ist als in einem (vollständig) gefüllten Zustand.

Eine Ausführungsform der Implantationshilfe zeichnet sich dadurch aus, dass das Lagerteil eine Aufnahme aufweist, die dazu vorgesehen und eingerichtet ist, das zu implantierende Reservoir vor seiner Implantierung zumindest teilweise in seinem Inneren aufzunehmen. Aus dieser Aufnahme ist das Reservoir dann mittels des Schlittens (vollständig) herausschiebbar. Eine solche Aufnahme umschließt das an der Implantationshilfe festgelegte Reservoir zumindest teilweise bzw. abschnittsweise und schützt es damit vor seiner Implantierung vor etwaigen Beschädigungen. Bevorzugt bildet das Lagerteil hierfür einem Abschnitt in der Art eines Gehäuses aus, dessen Wandungen einen Hohlraum zumindest teilweise umschließen, aus dem heraus das Reservoir mittels des Schlittens in der Ausbringungsrichtung ausgebracht werden kann. Ein solches Gehäuse oder Aufnahmeabschnitt des Lagerteils definiert somit eine das Reservoir schützende Aufnahme, in dem das Reservoir untergebracht werden kann.

Damit das Reservoir auch beim Ausschieben aus einer solchen Aufnahme zunächst in einer vorgegebenen Lage verbleibt und insbesondere ein flexibles oder elastisches Reservoir nicht unkontrolliert zusammengeschoben wird, ist in einer Ausführungsform vorgesehen, dass das wenigstens eine stabförmige Führungsmittel, an dem das Reservoir befestigbar ist (vollständig) aus der Aufnahme heraus verschiebbar ist. Ein Reservoir wird somit zusammen mit dem Führungsmittel aus der Aufnahme herausgeschoben und wird erst im Anschluss daran von dem Führungsmittel entfernt bzw. löst sich erst im Anschluss daran (zum Beispiel aufgrund seiner Elastizität automatisch) von dem außerhalb der Aufnahme liegenden Führungsmittel.

Alternativ oder ergänzend kann das Lagerteil eine Führung aufweisen, wobei die Führung eine Führungsbahn vorgibt, entlang der ein Teil des zu implantierenden Reservoirs während einer durch den Schlitten verursachten Verschiebung des Reservoirs gleitet. Auf diese Weise soll sichergestellt werden, dass zum Beispiel an dem Reservoir vorstehende Komponenten, wie zum Beispiel eine mit dem Reservoir verbundene Anschlussleitung für das Fluid, gleichermaßen zwangsgeführt und damit ohne die Gefahr einer Beschädigung bei dem Ausschiebevorgang mitverlagert werden. In einer bevorzugten Ausführungsform ist eine solche Führung als offener, längserstreckter Führungskanal an der Aufnahme oder in einer Seitenwand des sie definierenden Gehäuses oder Aufnahmeabschnitts vorgesehen. Ein solcher Führungskanal verläuft dann bevorzugt geradlinig und parallel zur Ausbringungsrichtung.

Zwar ist grundsätzlich auch eine automatisierte, das heißt, maschinelle Bedienung einer erfindungsgemäßen Implantationshilfe denkbar. Jedoch ist eine bevorzugte Ausgestaltung der Implantationshilfe für die manuelle Bedienung vorgesehen und eingerichtet und weist daher ein mit dem Schlitten verbundenes Griffteil auf, das über ein Kraftübertragungselement eine von einem Benutzer der Implantationshilfe auf das Griffteil einwirkende Kraft auf den Schlitten zu dessen Verstellung überträgt. Bei einem solchen Kraftübertragungselement kann es sich insbesondere um eine das Griffteil mit dem Schlitten verbindende Schubstange handeln. Die Schubstange ist hierbei innerhalb des Lagerteils verschieblich gelagert, zum Beispiel in einer Hülse oder Buchse des Lagerteils gleitend geführt.

Sofern eine Betätigungsrichtung des Kraftübertragungselements, in die eine Verstellkraft auf das Kraftübertragungselement zur Verstellung des Schlittens aufgebracht, und die Ausbringungsrichtung des Reservoirs und/oder die Verstellrichtung des Schlittens nicht miteinander übereinstimmen, sondern zum Beispiel winklig zueinander angeordnet sind, kann beispielsweise zwischen dem Kraftübertragungselement und dem Schlitten eine Gelenkanordnung vorgesehen sein, um eine auf das Kraftübertragungselement wirkende Kraft entsprechend umzulenken. Ein Operateur schiebt hierbei beispielsweise eine Schubstange zur Ausbringung des Reservoirs in einer Betätigungsrichtung. Eine Führungsbahn für den Schlitten verläuft jedoch nicht parallel, also winklig hierzu, so dass zwischen Schubstange und Schlitten z.B. eine Gelenkanordnung mit einem Kugelgelenk vorgesehen ist und eine Verschiebung der Schubstange in die Betätigungsrichtung eine Verschiebung des Schlittens (mit dem Reservoir) in einer hierzu unterschiedliche, winklig verlaufende Ausbringungsrichtung hervorruft.

Um zu verhindern, dass ein in der Implantationshilfe festgelegtes Reservoir unbeabsichtigt verlagert und zum Beispiel aus einer das Reservoir schützenden Aufnahme heraus geschoben wird, ist in einer Ausführungsform eine Sperre vorgesehen, die in einem Arretierzustand an der Implantationshilfe festlegbar ist. In diesem Arretierzustand verhindert die Sperre zumindest eine Verstellung des Schlittens in die Ausbringungsrichtung; sie blockiert folglich bis zu ihrem Entfernen oder Lösen eine Verstellung des Schlittens. Hierbei kann die Sperre insbesondere als separates Bauteil ausgebildet sein, das an der Implantationshilfe (zum Beispiel im Bereich eines Griffteils) lösbar befestigt und vollständig von dieser entfernbar ist, um eine Verstellung des Schlittens zu gestatten.

Im Zuge einer möglichst kostengünstigen Fertigung der Implantationshilfe ist vorgesehen, diese vollständig aus einem Kunststoffmaterial insbesondere einem biokompatiblen Kunststoffmaterial, wie zum Beispiel einem Polyethylen-Werkstoff herzustellen.

Ferner ist in einer Ausführungsform vorgesehen, dass die Implantationshilfe und/oder zumindest der Schlitten mehrteilig ausgebildet sind, wobei die einzelnen Bauteile ausschließlich zusammengesteckt sind. Es sind mit anderen Worten keine weiteren separaten Befestigungsmittel, wie Schrauben, Klemmen oder Klammern, vorgesehen, um die einzelnen Bauteile (dauerhaft) miteinander zu verbinden. Vielmehr werden die einzelnen (Kunststoff-) Bauteile jeweils mit Steckzapfen oder Steckhülsen/-buchsen ausgebildet und durch Einstecken eines Bauteils mit einem Steckzapfen in ein Bauteil mit einer Steckhülse bzw. -buchse kraftschlüssig miteinander verbunden. Dies gestattet eine äußerst kostengünstige Fertigung und reduziert zudem das Gewicht der Implantationshilfe.

Ein weiterer Aspekt der vorliegenden Erfindung ist eine Implantationseinheit nach dem Anspruch 11, die aus einer erfindungsgemäßen Implantationshilfe und einem implantierbaren, befüllbaren Reservoir einer Penisprothese gewonnen wird. Eine derartige Implantationseinheit stellt eine vormontierte, auslieferbare und zur sofortigen Verwendung vorgesehene Einheit dar, an der bereits ein zu implantierendes Implantat angebracht bzw. untergebracht ist. Das Reservoir ist demgemäß an der Implantationshilfe lösbar festgelegt, die unter anderem zusammen mit dem Reservoir die Implantationseinheit bildet. Mittels des Schlittens ist dann das Reservoir zur Implantierung relativ zu dem Lagerteil der Implantationshilfe in die Ausbringungsrichtung verschiebbar.

Demgemäß ist ein weiterer Aspekt der vorliegenden Erfindung ein Verfahren zur Herstellung einer Implantationseinheit mit implantierbarem und befüllbarem Reservoir einer Penisprothese gemäß dem Anspruch 12.

Ein solches erfindungsgemäßes Verfahren weist hierbei die folgenden Schritte auf:
- Bereitstellung einer Implantationshilfe mit einem Lagerteil, an dem ein zu implantierendes Reservoir festlegbar ist und das einen relativ zu dem Lagerteil entlang einer Ausbringungsrichtung verstellbaren Schlitten aufweist;
- Verstellung des Schlittens in Ausbringungsrichtung;
- Anbringung des Reservoirs an dem Schlitten;
- Verstellung des Schlittens mit daran angebrachtem Reservoir entgegen der Ausbringungsrichtung.

Die erstmalige Verstellung des Schlittens in Ausbringungsrichtung erfolgt in Anlehnung an die bereits zuvor dargelegten Ausführungsbeispiele zum Beispiel derart, dass zumindest ein Teil des Schlittens von einer Aufnahme des Lagerteils vorsteht, wobei dann die Verstellung des Schlittens mit daran angebrachtem Reservoir entgegen der Ausbringungsrichtung demgemäß dazu führt, dass das Reservoir zumindest teilweise im Inneren der Aufnahme untergebracht wird.

Das Reservoir ist ferner lösbar an dem Schlitten befestigt, so dass das Reservoir zu seiner Implantierung wieder ohne Weiteres von dem (ausgefahrenen) Schlitten entfernbar ist.

In einem bevorzugten Ausführungsbeispiel weist das Reservoir eine zumindest teilweise flexible oder sogar elastische Hülle auf und wenigstens ein flexibler (elastischer) Abschnitt der Hülle wird zur Festlegung des Reservoirs an dem Schlitten verformt, insbesondere gewickelt oder gefaltet. Hierfür kann der Schlitten beispielsweise ein (stabförmiges) Führungsmittel aufweisen, um das (herum) ein flexibler oder sogar elastischer Abschnitt der Hülle des Reservoirs gewickelt oder gefaltet wird.

Bei elastischer oder zumindest teilelastischer Ausbildung des Reservoirs und Einfahren des Schlittens in eine das Reservoir zumindest teilweise oder vollständig umschließenden Aufnahme kann erreicht werden, dass das elastisch verformte und damit vorzugsweise kompaktere Reservoir in der Aufnahme untergebracht ist und sich erst nach vollständigem Ausbringen aus der Aufnahme wieder entfalten und somit in seine ursprüngliche Form zurückkehren kann. Dies hat ferner den Vorteil, dass ein manuelles Lösen oder Entfernen des ausgeschobenen Reservoirs von der Implantationshilfe oder seinem Schlitten oder dem wenigstens einen Führungsmittel des Schlittens nicht notwendig ist, sondern dass sich das Reservoir aufgrund seiner Elastizität selbsttätig löst, sobald es (vollständig) aus der Implantationshilfe ausgebracht wurde und es die Aufnahme nicht mehr an der Rückkehr zu seiner ursprünglichen Form hindert.

Weitere mögliche Ausgestaltungsvarianten sind auch durch die Unteransprüche gegeben.

Darüber hinaus werden weitere Merkmale und Vorteile der Erfindung anhand der nachfolgenden Beschreibung eines Ausführungsbeispiels deutlich werden. Dabei stellen für ein Ausführungsbeispiel einer Implantationshilfe oder einer Implantationseinheit als vorteilhaft herausgestellte Merkmale ebenfalls auf vorteilhafte Merkmale für ein Verfahren zur Herstellung einer Implantationseinheit dar und umgekehrt.

Es zeigen:
- Figur 1: eine aus dem Stand der Technik bekannte Penisprothese im implantierten Zustand;
- Figuren 2A-2B: ein zu implantierendes, mit Fluid befüllbares Reservoir einer Penisprothese im unbefüllten Zustand in verschiedenen Ansichten;
- Figuren 3A-3B: das Reservoir der Figuren 2A und 2B in befülltem Zustand;
- Figur 4A: ein Ausführungsbeispiel einer erfindungsgemäßen Implantationseinheit mit erfindungsgemäß ausgestalteter Implantationshilfe, bei der ein verstellbarer Schlitten, an dem das zu implantierende Reservoir festgelegt ist, in eingefahrenem Zustand vorliegt;
- Figur 4B: die Implantationseinheit der Figur 4A mit ausgefahrenem Schlitten;
- Figuren 4C-4D: Schnittansichten der Implantationseinheit gemäß Figur 4A;
- Figuren 5A-5C: Einzelansichten des Schlittens mit daran angebrachtem Reservoir;
- Figuren 6A-6C: Einzelansichten des Schlittens ohne Reservoir;
- Figur 7: Einzeldarstellung eines von zwei Führungsmitteln des Schlittens;
- Figur 8: Einzeldarstellung eines Verbindungsstücks des Schlittens, das die beiden Führungsmittel trägt;
- Figuren 9A-9B: Einzeldarstellungen eines Steckverbinders des Schlittens, der das Verbindungsstück des Schlittens mit einem Kraftübertragungselement verbindet;
- Figur 10: Einzeldarstellung eines als Schubstange ausgebildeten Kraftübertragungselements;
- Figuren 11A-11C: Einzeldarstellungen eines Lagerteils der Implantationshilfe, in dem ein zu implantierendes Reservoir im eingefahrenen Zustand des Schlittens geschützt in einer Aufnahme untergebracht werden kann;
- Figur 12: in perspektivischer Ansicht eine Ausführungsform einer Sperre, mittels der entsprechend dem Ausführungsbeispiel der Figur 4A ein Ausfahren des Schlittens verhindert werden kann.

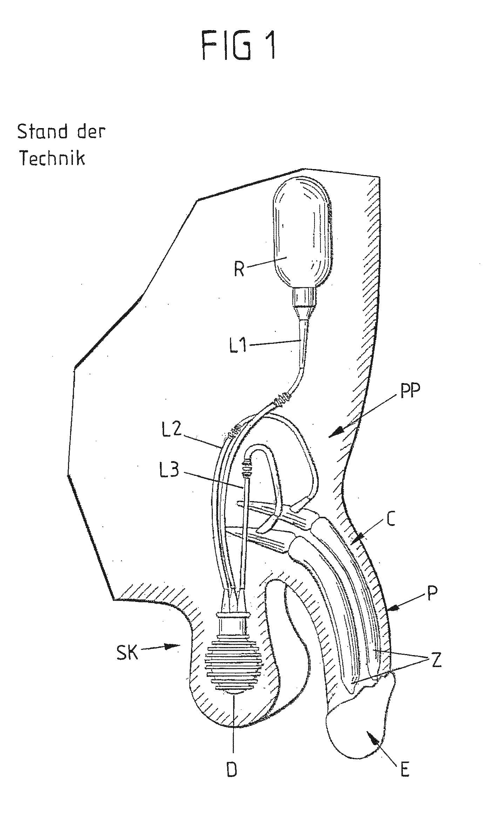

Die Figur 1 zeigt eine Penisprothese PP, wie sie zum Beispiel aus der US-A 4,566,446 bekannt ist, im implantierten Zustand.

Die Penisprothese PP weist unter anderem zwei in das Corpus cavernosum penis C, also den Penisschwellkörper, eines Penis P eines Patienten implantierte Zylinder Z auf. Diese Zylinder Z erstrecken sich von der Glans penis E des Penis P in Richtung des Beckens des Patienten. Sie sind aus einem flexiblen Material gefertigt und mit einem Fluid, üblicherweise eine Kochsalzlösung, mittels einer im Skrotum SK des Patienten untergebrachten Pumpeinheit D befüllbar, um eine Erektion des Penis P zu erzeugen. Das Fluid gelangt hierbei über je eine Leitung L2 oder L3 von der Pumpeinheit D zu dem jeweiligen Zylinder Z.

Das Fluid zur Befüllung der Zylinder Z wird in einem Reservoir R vorgehalten, aus dem das Fluid über eine Leitung L1 bei Bedarf zur Pumpeinheit D gelangt und in die Zylinder Z gepumpt werden kann. Sofern der Patient wieder in den nicht-erigierten Zustand wechseln möchte, wird das Fluid von den beiden Zylindern Z wieder in das Reservoir R gepumpt. Hierzu weist die Pumpeinheit D wenigstens ein betätigbares Ventil auf, das ein Pumpen der Flüssigkeit zurück in das Reservoir R gestattet.

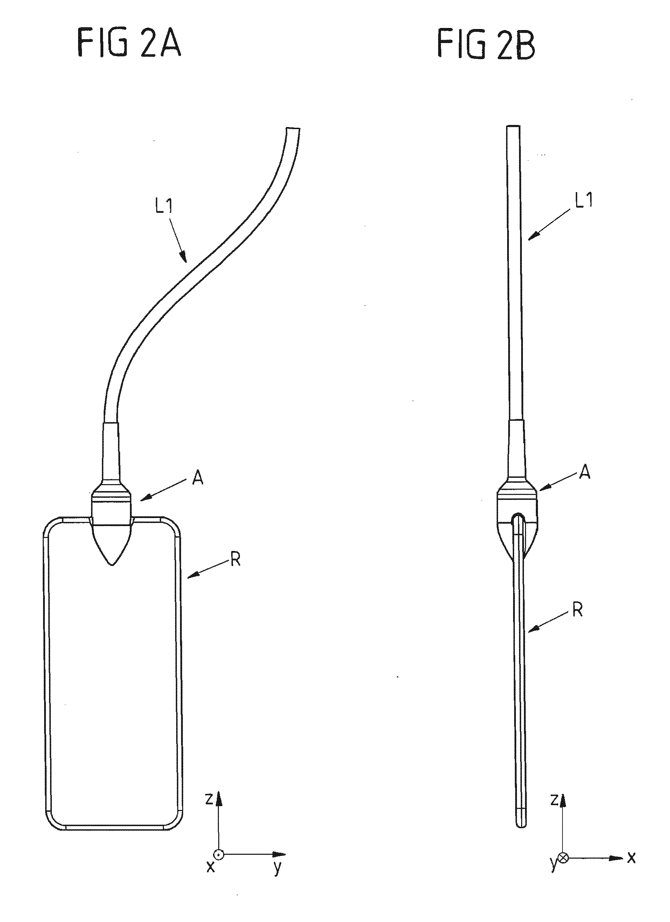

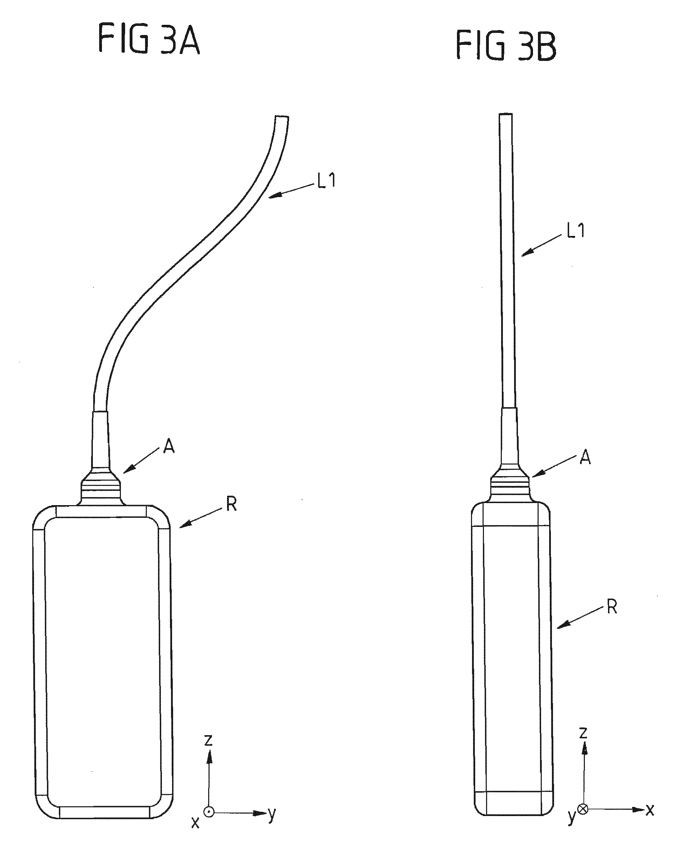

Ein Reservoir R der Penisprothese PP, das in den Figuren 2A-2B und 3A-3B in Einzeldarstellungen vergrößert dargestellt ist, ist üblicherweise in einem unbefüllten oder entleerten Zustand im Unterbauch des Patienten zu implantieren. Hierbei sind an dem zu implantierenden Reservoir R auch bereits ein Adapterstück A zur fluidtechnischen Verbindung des Reservoirs R mit der (Anschluss-) Leitung L1 vorgesehen Das Adapterstück A ist hierbei üblicherweise notwendig, um einen Durchmesserunterschied zwischen dem Durchmesser der Leitung L1 und einer durch den Herstellungsprozess des Reservoirs R bedingten, größeren Öffnung des Reservoirs R für das Fluid auszugleichen.

Diese Leitung L1 ist somit gleichfalls bereits an dem Reservoir R vormontiert oder damit ausgeformt, so dass sie als ein Teil des Reservoirs R zusammen mit dem Reservoir R im Rahmen eines operativen Eingriffs zu platzieren ist.

Das Reservoir R wird aus einer flexiblen oder elastischen Hülle gebildet, die sich bei Befüllung mit Fluid ausgehend von einem in den Figuren 2A und 2B gezeigten unbefüllten Zustand ausdehnen kann. Ein entsprechender gefüllter Zustand des Reservoirs R mit gedehnter oder gespannter Hülle ist in den Figuren 3A und 3B gezeigt. Die hier gezeigte quaderförmige Ausbildung des Reservoirs R ist selbstverständlich nur beispielhaft. Es sind selbstverständlich auch andere Formen für ein Reservoir R denkbar, wie zum Beispiel eine sphärische oder ballonartige oder zylindrische Form.

Bei der Implantation einer Penisprothese PP stellt sich unter anderem die bestimmungsgemäße Platzierung des Reservoirs R im Unterleib des Patienten als schwierig dar. So erfolgt die Platzierung des Reservoirs R regelmäßig hinter dem Beckenknochen des Patienten, so dass der Operateur während des Eingriffs keinen unmittelbaren Sichtkontakt zu dem bereits in den Körper des Patienten eingebrachten Reservoir R hat. Die Platzierung erfolgt somit nahezu blind. Hierbei erschwert insbesondere die flexible Struktur des Reservoirs R eine bestimmungsgemäße Platzierung und Ausrichtung des Reservoirs R.

Zudem wird das Reservoir R, das üblicherweise nur geringe Wandstärken im Bereich von 0,5 bis 2 mm aufweist, dem Operateur unmittelbar vor dem Eingriff lose übergeben, so dass die Gefahr besteht, dass das Reservoir R vor seiner Implantierung beschädigt wird. Es muss daher mit größter Vorsicht gehandhabt werden.

Mit einer erfindungsgemäßen Implantationshilfe, die auch zusammen mit einem daran festgelegten Reservoir R eine dem Operateur zur Verfügung stehende Implantationseinheit bilden kann, können die genannten Nachteile vermieden oder zumindest gemindert werden und es lässt sich der operative Eingriff zur Implantierung des Reservoirs R optimieren.

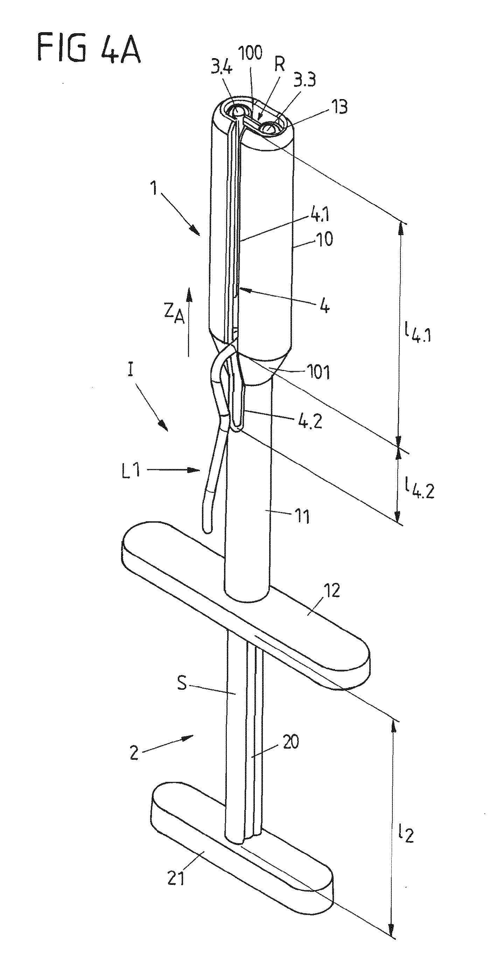

Die Figur 4A zeigt ein Ausführungsbeispiel einer erfindungsgemäßen Implantationshilfe I, die zusammen mit einem daran festgelegten Reservoir R eine vormontierte Implantationseinheit bildet, die als medizinisches Instrument dem Operateur zur Verfügung gestellt werden kann und von der das zu implantierende Reservoir R in einer vorgegebenen Lage entfernbar ist.

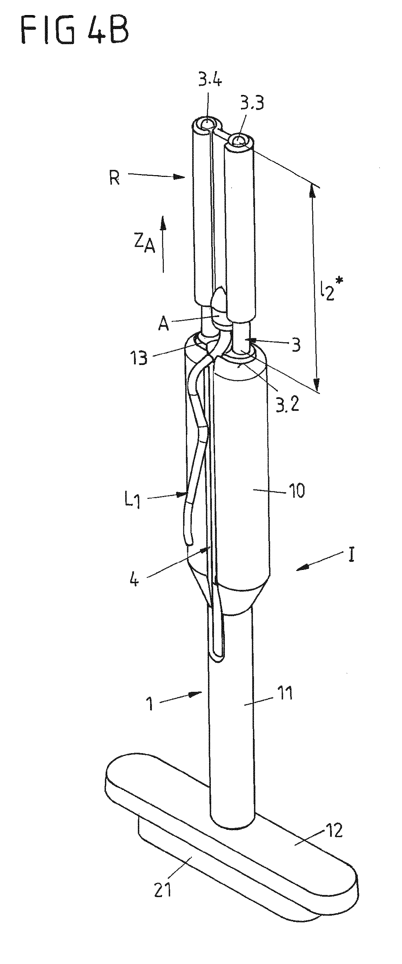

Die Implantationshilfe I weist ein Lagerteil 1 mit einer Aufnahme 100 auf, in der ein Reservoir R vorgehalten werden kann und in der ein Schlitten 3 (vergleiche Figur 4B) verschieblich gelagert ist.

Die gehäuseartige Aufnahme 100 des Lagerteils 1 ist hierbei in einem vorderen Aufnahmeabschnitt 10 des Lagerteils 1 vorgesehen und weist einen symmetrischen Querschnitt auf. Dieser symmetrische Querschnitt setzt sich aus einem Rechteck und zwei Halbkreise zusammen. Dabei schließt sich an das mittige Rechteckt an dessen sich gegenüber liegenden kurzen Seiten jeweils ein Halbkreis an, so dass der Querschnitt ellipsenartig erscheint.

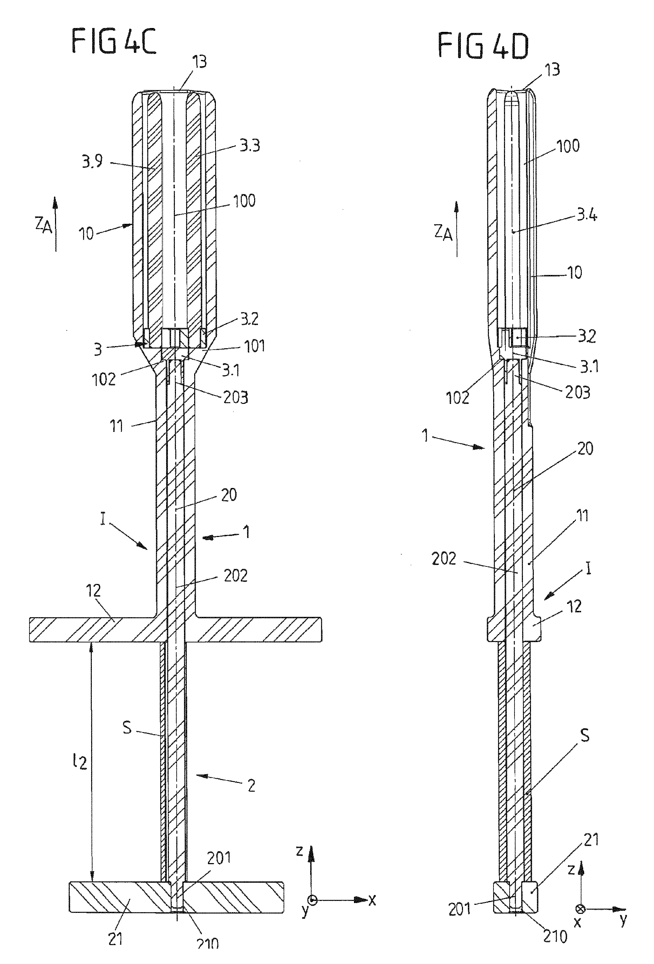

Der von dem Aufnahmeabschnitt 10 als Aufnahme 100 berandete Hohlraum mit dem erläuterten Querschnitt mündet über einen sich verjüngenden Verbindungsbereich 101 in einen Lagerungsabschnitt 11 des Lagerteils 1. Am Ende des Lagerungsabschnitts 11 befindet sich das Zwischenstück 12, das einstückig mit dem Lagerungsabschnitt 11 ausgebildet ist. Ferner ist der Lagerungsabschnitt 11 gleichfalls hohl und weist in seinem Inneren eine Durchgangsbohrung auf, die als Führung für ein Kraftübertragungselement in Form einer Schubstange 20 dient. Dies ist insbesondere anhand der Schnittdarstellungen der Figuren 4C und 4D gut ersichtlich.

Die Länge des Lagerungsabschnitts 11 sowie die Länge der Schubstange 20 können im Übrigen angepasst, das heißt, gegenüber dem dargestellten Ausführungsbeispiel kürzer oder länger sein, um mittels einer Implantationshilfe I ein Reservoir R an einer anderen bestimmungsgemäßen Stelle innerhalb eines Körpers zu platzieren. So kann insbesondere vorgesehen sein, dass die Schubstange 20 und der Lagerungsabschnitt 11 mit dem Zwischenstück 12 auswechselbar ausgebildet sind. Hierfür kann beispielsweise an dem Aufnahmeabschnitt 10 ein Außengewinde ausgebildet sein, auf das ein Innengewinde des Lagerungsabschnitts 11 aufgeschraubt ist. Demgegenüber ist in dem gezeigten Ausführungsbeispiel der Aufnahmeabschnitt 10 einstückig mit dem Lagerungsabschnitt 11 und dem Zwischenstück 12 ausgebildet.

Der Schlitten 3 ist aus einer Ruhelage heraus, in der das Reservoir R in der Aufnahme 100 untergebracht ist, in eine Ausbringungsrichtung Z_{A} verstellbar, so dass das an dem Schlitten 3 angebrachte Reservoir R aus einer in Ausbringungsrichtung Z_{A} liegenden Ausbringungsöffnung 13 aus der Aufnahme 100 der Implantationshilfe I ausgeschoben wird.

Die Betätigung des Schlittens 3 erfolgt dabei über ein Griffteil 2 der Implantationshilfe I. Das Griffteil 2 ist relativ zu dem Lagerteil 1 verschieblich geführt und greift über ein Kraftübertragungselement in Form der starren Schubstange 20 an dem Schlitten 3 des Lagerteils 1 an. Damit wird eine auf die Schubstange 20 aufgebrachte Verstellbewegung in Ausbringungsrichtung Z_{A} auf den Schlitten 3 übertragen, so dass der Schlitten 3 in Ausbringungsrichtung Z_{A} verschoben wird.

Zur einfacheren, manuellen Bedienung der Implantationshilfe I bzw. der Implantationseinheit I, R weist das Griffteil 2 an dem in der Ruhelage des Schlittens 3 von dem Lagerteil 1 vorstehenden Ende der Schubstange 20 ein Griffelement 21 auf. An diesem Griffelement 21 kann ein Operateur angreifen, um den Schlitten 3 zu verstellen und das Reservoir R aus der Aufnahme 100 auszuschieben.

Das Griffelement 21 ist ferner zum Ausschieben des Reservoirs R in Ausbringungsrichtung Z_{A} auf ein Zwischenstück 12 des Lagerteils 1 zu verstellbar. Dieses Zwischenstück 12 erstreckt sich (wie das Griffelement 21) im Wesentlichen quer zur Längserstreckungsrichtung der Schubstange 20 und bildet ein Ende des hohlen Lagerteils 1, das von den Fingern eines Operateurs umgriffen werden kann, um mit dem Daumen oder Handballen das Griffelement 21 in Richtung des Zwischenstücks 12 zu drücken. Die Implantationshilfe I bzw. die aus Implantationshilfe I und Reservoir R gebildete Implantationseinheit ist somit einhändig bedienbar, um ein in der Aufnahme 100 eingesetztes Reservoir R auszubringen und bestimmungsgemäß zu platzieren.

Um ein unabsichtliches Verlagern oder Verstellen des Schlittens 3 aus seiner Ruhelage bzw. seinem eingefahrenen Zustand (Figur 4A) in die Ausbringungsrichtung Z_{A} zu verhindern, ist eine längserstreckte Sperre S vorgesehen. Die Sperre S wird in dem vorliegenden Ausführungsbeispiel durch eine geschlitzte, im Querschnitt C-förmige Hülse gebildet. Die längserstreckte Sperre S weist dabei eine Länge l₂ auf, die im Wesentlichen dem maximal möglichen Abstand des Griffelements 21 von dem Zwischenstück 12 entspricht. Durch Ihre geschlitzte Ausführung ist die Sperre S auf den aus dem Lagerteil 1 hervorstehenden Abschnitt der Schubstange 20 quer zu dessen Längserstreckung aufsteckbar. Durch die durchgängige Schlitzung der Sperre S ist diese ferner so elastisch, dass sie auf die Schubstange 20 aufgesteckt werden kann und nach ihrem Aufstecken auf die Schubstange 20 derart formschlüssig mit dieser verbunden ist, dass die Sperre S nicht selbsttätig, das heißt, ohne Krafteinwirkung von außen, von der Schubstange 20 wieder entfernt werden kann.

Die Sperre S weist einen größeren Querschnitt auf als eine zylindrische Führung in dem Lagerteil 1, in der die Schubstange 20 gleitend geführt ist, so dass eine Stirnseite der Sperre S an einer dem Griffteil 21 zugewandten Seite des Zwischenstücks 12 anliegt oder anstößt, wenn in dem in der Figur 4A gezeigten Arretierzustand der Sperre S eine Verstellkraft in Ausbringungsrichtung Z_{A} auf das Griffelement 21 bzw. den Schlitten 3 aufgebracht wird. Gleichzeitig liegt die andere, gegenüberliegende Stirnseite der Sperre S an einer dem Zwischenstück 12 zugewandten Seite des Griffelements 21 an, so dass die in Längsrichtung starre Sperre S eine Verstellung des Griffelements 21 in Richtung auf das Zwischenstück 12 und damit in Ausbringungsrichtung Z_{A} blockiert.

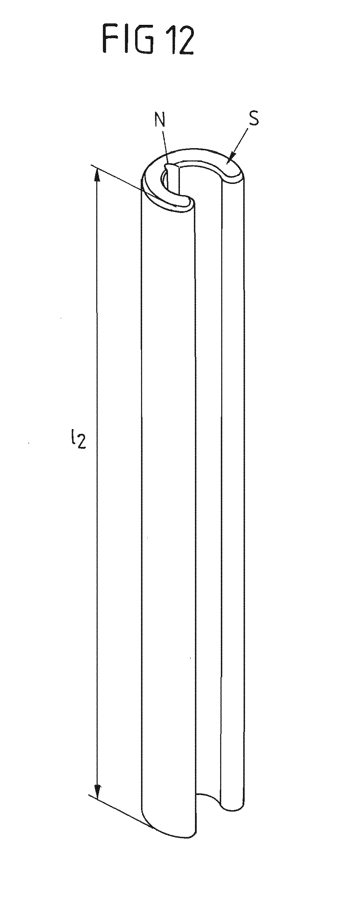

Zum einfachen Ablösen der Sperre S weist diese in der Mitte des C-förmigen Profils eine durchgehende Längsnut entlang der Längserstreckung der hülsenartigen Sperre S auf, die ein Aufweiten des Profils bzw. der Hülse erleichtert (vgl. auch nach stehend noch erläuterte Figur 12).

Es ist selbstverständlich denkbar, die Sperre S abweichend von der dargestellten Ausführungsform auszugestalten. Dabei ist neben der Verwendung alternativer Profile denkbar, eine Sperrfunktion unmittelbar in die Schubstange 20 zu integrieren, indem das Profil der Schubstange 20 nur in einer bestimmten Ausrichtung in die Führung im Inneren des Lagerteils 1 passt. Beispielsweise kann durch Ausbildung der Schubstange 20 mit einem im eingefahrenen Zustand des Schlittens 3 aus dem Lagerteil 1 vorstehenden Abschnitt, der einen quadratischen Querschnitt aufweist, und gleichzeitiger drehbarer Lagerung der Schubstange 20 um eine zu ihrer Längserstreckungsrichtung parallele Achse verhindert werden, dass die Schubstange 20 unbeabsichtigt verstellt wird. Hierfür würde ferner auch die Führung für die Schubstange 20 innerhalb des Lagerteils 1 einen mit dem quadratischen Querschnitt des heraus- oder vorstehenden Schubstangenabschnitts korrespondierenden Querschnitt aufweisen und die Schubstange 20 in der in der Figur 4A dargestellten Lage (dem eingefahrenen Zustand des Schlittens 3) verdrehbar sein, ggf. durch das Vorsehen eines Drehlagers an der Anbindungsstelle der Schubstange 20 an dem Schlitten 3. So ließe sich die Schubstange 20 erst wieder zur Ausbringung des Schlittens 3 verschieben, wenn der Querschnitt ihres vorstehenden Abschnitts mit dem Querschnitt der in dem Lagerteil 1 vorgesehenen Führung fluchtet und der Schubstangenabschnitt hierin eingeführt werden kann.

Alternativ kann auch eine quer zur Längserstreckungsrichtung der Schubstange 20 verlaufende Bohrung in der Schubstange 20 vorgesehen sein, in die ein lösbarer Sperrstift als Sperre S derart eingesteckt ist, dass er an dem Zwischenstück 12 anschlagen kann. Derart verhindert ein solcher Sperrstift eine (weitere) Verstellung der Schubstange 20 relativ zu dem Lagerteil 1 in Ausbringungsrichtung Z_{A}.

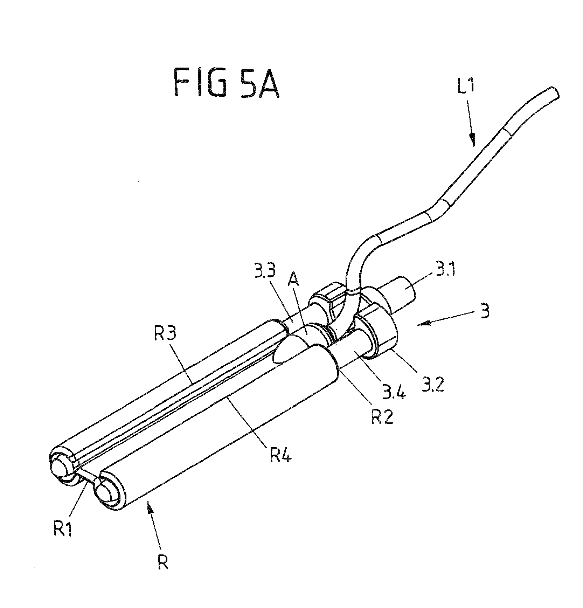

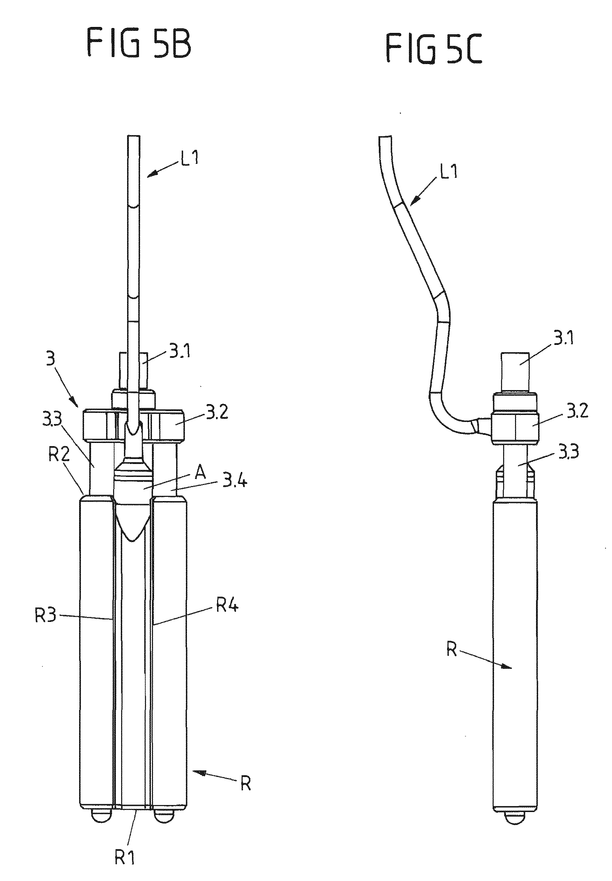

Dem Vorsehen einer Sperre S an der Implantationshilfe I kommt in dem vorliegenden Ausführungsbeispiel auch deshalb eine große Bedeutung zu, da das Reservoir R lose an dem Schlitten 3 ohne zusätzliche Befestigungsmittel angebracht ist. So ist das Reservoir R, wie in den Detaildarstellungen der Figuren 5A bis 5C noch näher veranschaulicht ist, lediglich um zwei stabförmige Führungsmittel 3.3 und 3.4 des Schlittens 3 gewickelt oder gefaltet, so dass die um diese Führungsmittel 3.3, 3.4 gewickelten oder gefalteten elastischen Abschnitte der Hülle des Reservoirs R sich selbsttätig von den Führungsmitteln 3.3, 3.4 abwickeln bzw. entfalten, sobald sie nicht länger von den sie im eingefahrenen Zustand des Schlittens 3 umgebenden Wandungen der Aufnahme 100 daran gehindert werden. Erst wenn also das Griffteil 2 bzw. sein Griffelement 21 (nach dem vollständigen Entfernen der Sperre S) um die Länge l₂ und damit bis zum Anschlag an das Zwischenstück 12 in Ausbringungsrichtung Z_{A} verschoben wurde, so dass die stabförmigen (zylindrischen) Führungsmittel 3.3, 3.4 des Schlittens 3 mit ihrer vollständigen Länge l₂* aus der Aufnahme 100 hervorstehen, wird das Reservoir R freigegeben und kann sich vollständig von den Führungsmitteln 3.3, 3.4 abwickeln oder entfalten, um seine in den Figuren 2A und 2B gezeigte ursprüngliche Form anzunehmen.

An der Wandung der Aufnahme 100 beziehungsweise in dem Aufnahmeabschnitt 10 ist weiterhin eine Führung in Form eines längserstreckten und parallel zu der Ausbringungsrichtung Z_{A} verlaufenden Führungskanals 4 vorgesehen. Der Führungskanal 4 verläuft mittig auf einer ebenen Seite des Aufnahmeabschnitts 10. Dieser Führungskanal 4 gibt eine Führungsbahn für einen Teil des Reservoirs R vor, der nicht im Inneren der Aufnahme 100 aufgenommen ist, hier für die Leitung L1. Über den Führungskanal 4 wird somit eine Führungsbahn für die (Anschluss-)Leitung L1 des Reservoirs R vorgegeben, entlang derer die Leitung L1 bei einem Ausbringen des Reservoirs R aus der Aufnahme 100 zwangsgeführt ist. Hierdurch wird ein Knicken oder Verhacken der Leitung L1 während des Ausschiebevorgangs verhindert.

Damit die Leitung L1 gezielt zusammen mit dem Reservoir R ausgebracht werden kann, verläuft der Führungskanal als in Ausbringungsrichtung Z_{A} Schlitz oder nach außen offene Längsnut an dem Aufnahmeabschnitt 10. Der Führungskanal 4 mündet damit in die Ausbringungsöffnung 13 der Aufnahme 100 an der bei bestimmungsgemäßem Gebrauch dem Körper zugewandten Vorderseite des Lagerteils 1.

Der Führungskanal 4 untergliedert sich hierbei in zwei Führungsabschnitte 4.1 und 4.2. Dabei entspricht die Länge des Führungsabschnitts 4.1 der Gesamtlänge l_{4.1} des Aufnahmeabschnittes 10, so dass der zweite Führungsabschnitt 4.2 mit seiner Länge l_{4.2} bis in den Lagerungsabschnitt 11 hineinreicht. Hierdurch wird erreicht, dass bei vollständig eingefahrenem Schlitten 3 und darin festgelegtem Reservoir R, dessen Länge hier im Wesentlichen der Länge der Führungsmittel 3.3. und 3.4 entspricht, dennoch ein Teil der Leitung L1 ohne Verknicken in dem Führungskanal 4 untergebracht ist.

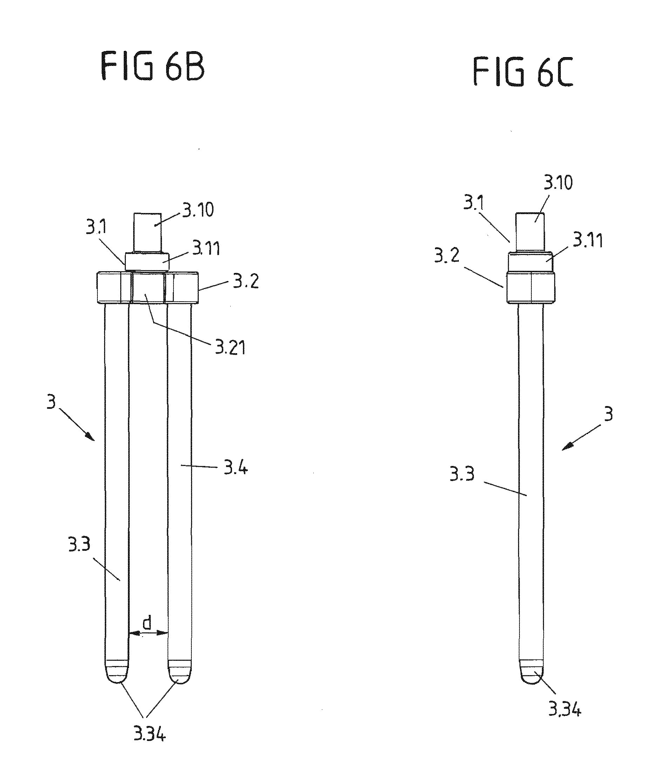

In den Figuren 5A - 5C ist nun der Schlitten 3 mit daran angebrachtem Reservoir R in verschiedenen Ansichten im Detail dargestellt. Der Schlitten 3 bildet mit seinen parallel zueinander verlaufenden, stabförmigen Führungsmitteln 3.3 und 3.4, die in einem Abstand d (vergleiche Figur 6B) beabstandet zueinander angeordnet sind, ein (starres) Gerüst für das daran festzulegende Reservoir R. An diesem Gerüst ist das Reservoir durch Umwickeln oder Umfalten zweier sich quer zur Längserstreckungsrichtung der Führungsmittel 3.3 und 3.4 gegenüberliegender Längsränder R3 und R4 lösbar angebracht. Die beiden Längsränder R3, R4 sind dabei jeweils aufeinander zu gewickelt oder gefaltet, so dass der an einem Querrand R2 des Reservoirs R angebrachte Adapter A zwischen den Führungsmitteln 3.3 und 3.4 ruht und damit dem der Ausbringungsöffnung 13 zugewandten Querrand R1 im Wesentlichen parallel gegenüberliegend. Vorliegend entspricht die Länge l₂* der Führungs mittel 3.3 und 3.4 im Wesentlichen der Länge des Reservoirs R bzw. seiner Längsränder R3 und R4, so dass der Querrand R1 nahezu mit dem vorderen Ende der Führungsmittel 3.3, 3.4 abschließt.

Ein Abwickeln oder Entfalten des an dem durch die beiden Führungsmittel 3.3 und 3.4 gebildeten Gerüsts wird im eingefahrenen Zustand des Schlittens 3 durch die Wandungen der Aufnahme 100 verhindert. Die Wandungen der Aufnahme 100 beranden die Führungsmittel 3.3 und 3.4 im eingefahrenen Zustand des Schlittens 3 in derart geringem Abstand, dass dem Reservoir R kein ausreichender Platz zum Abwickeln bzw. Entfalten zur Verfügung steht. Darüber hinaus ist das Material der Wandungen der Aufnahme 100 derart gewählt, dass das an dem Schlitten 3 angebrachte Reservoir R entlang der Wandungen gleiten kann, um möglichst reibungsarm aus der Aufnahme 100 heraus geschoben werden zu können. Die Aufnahme 100 und der Schlitten 3 sind somit derart aufeinander abgestimmt, dass der Schlitten 3 (mit den Führungsmitteln 3.3, 3.4) und das daran angebrachte, entleerte und gefaltete oder gewickelte Reservoir R in der Aufnahme 100 Platz finden und beweglich sind, ohne dass sich die durch Wickeln oder Falten vorgegebene Lage des Reservoirs R bis zur vollständigen Ausbringung aus der Aufnahme 100 (wesentlich) verändern kann.

Der Schlitten 3 ist vorliegend aus mehreren, zusammengesteckten Teilen oder Komponenten 3.1, 3.2, 3.3 und 3.4 aufgebaut. Neben den Führungsmitteln 3.3 und 3.4 handelt es sich hierbei um ein Verbindungsstück 3.2, das die beiden Führungsmittel 3.3 und 3.4 trägt und in das die beiden Führungsmittel 3.3 und 3.4 eingesteckt sind, sowie um einen Steckverbinder 3.1, der seinerseits in das Verbindungsstück 3.2 eingesteckt ist und in den die Schubstange 20 eingesteckt werden kann. Der Steckverbinder 3.1 fungiert hier somit als ein Schubstangenadapter, über den die Schubstange 20 mit den anderen Teilen des Schlittens 3 verbunden ist.

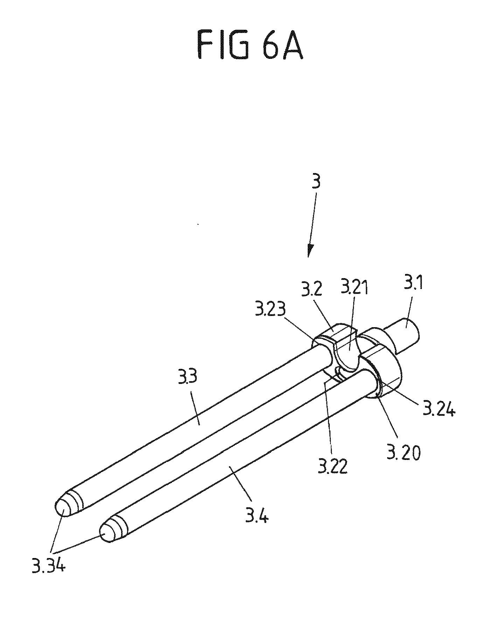

Der Schlitten 3 und seine Komponenten 3.1, 3.2, 3.3 und 3.4 sind den Einzeldarstellungen der Figuren 6A - 6C sowie 7, 8 und 9A - 9B vergrößert dargestellt. Dabei zeigen die Figuren 6A - 6C in mit den Figuren 5A - 5C übereinstimmenden Ansichten den zusammengebauten Schlitten 3 ohne ein daran festgelegtes Reservoir R, während in den übrigen genannten Figuren die den Schlitten 3 bildenden Komponenten in Einzeldarstellungen gezeigt sind.

Wie in der Zusammenschau der genannten Figuren gut ersichtlich ist, sind die einzelnen Komponenten des Schlittens (Steckverbinder 3.1, Verbindungsstück 3.2 und Führungsmittel 3.3, 3.4) im vorliegenden Ausführungsbeispiel aus Kunststoff gefertigt und werden lediglich zusammengesteckt, so dass für ihr Verbindung jeweils keine zusätzlichen Befestigungsmittel, wie zum Beispiel Schrauben oder Nieten, nötig sind.

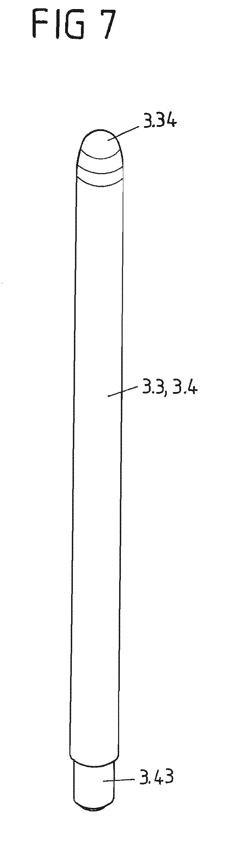

So weisen die Führungsmittel 3.3 und 3.4 jeweils an einem Ende einen stiftförmigen Absatz 3.43 auf, der in eine von zwei an dem Verbindungsstück 3.2 vorgesehenen zylindrischen Stecköffnungen 3.23 und 3.24 eingeführt werden kann, um ein Führungsmittel 3.3, 3.4 an dem Verbindungsstück 3.2 festzulegen. Wie in der Detailansicht der Führungsmittel 3.3, 3.4 der Figur 7 ferner ersichtlich ist, weist ein Führungsmittel 3.3, 3.4 an einem anderen, dem stiftförmigen Absatz 3.43 gegenüberliegenden Ende einen konischen, abgerundeten Endabschnitt 3.34 auf. Dieser konische, abgerundete Endabschnitt 3.34 vermindert das Risiko, dass beim Ausbringen des Reservoirs R aus der Aufnahme 100 ein Führungsmittel 3.3 oder 3.4 Gewebe des Patienten beschädigt.

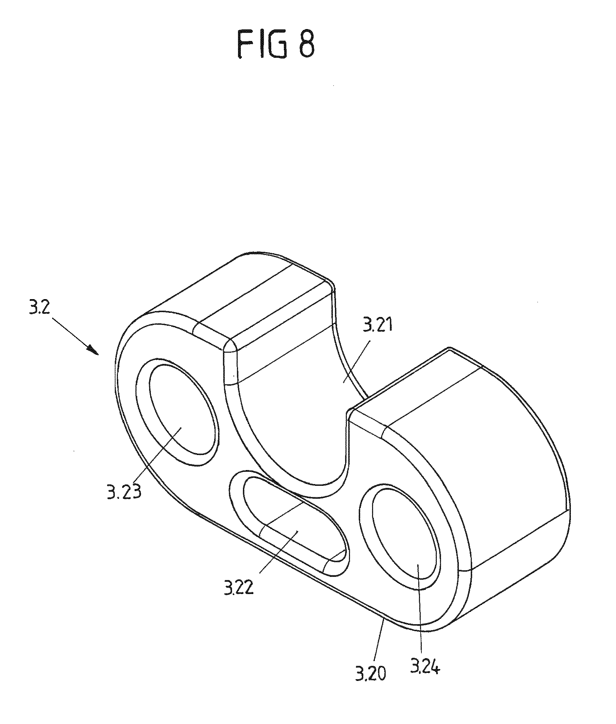

Das Verbindungsstück 3.2, das in der Figur 8 einzeln dargestellt ist, weist eine C-förmige Grundfläche 3.20 auf. Eine Aussparung 3.21 ist mittig an dem Verbindungsstück 3.2 vorgesehen. Die Aussparung 3.21 weist dabei die Form eines in der Längsachse halbierten Rohrstücks auf und ist somit kanalartig ausgebildet. Die Aussparung 3.21 dient der Aufnahme des Adapters A und/oder der von dem Reservoir R abgehenden (Anschluss-)Leitung L1, wenn das Reservoir R entsprechend den Figuren 5A - 5C an dem Schlitten 3 angebracht ist. Über die Aussparung 3.21 kann folglich auch der Adapter A beziehungsweise ein Stück der Leitung L1 an dem Schlitten 3 untergebracht oder gelagert werden.

Ferner bildet das Verbindungsstück 3.2 abgerundete Wangen oder Flanken aus, die durch die mittige Aussparung 3.21 voneinander räumlich getrennt sind. In diesen Flanken oder Wangen ist je eine der beiden zylindrischen Stecköffnungen 3.23, 3.24 ausgebildet. Die Wangen oder Flanken des Verbindungsstücks 3.2 liegen im eingebauten Zustand des Schlittens 3 in dem Lagerteil 1 an den Innenseiten der Wandungen der Aufnahme 100 an. Dabei ist das Verbindungsstück 3.2 so dimensioniert, dass es mit seinen Wangen oder Flanken entlang der Innenseiten der Wandungen der Aufnahme 100 gleiten kann und durch diese zwangsgeführt ist. Darüber hinaus ist der Schlitten 3 über das Verbindungsstück 3.2 und seine Wangen oder Flanken derart in der Aufnahme 100 gelagert, dass eine Verdrehung des Verbindungsstücks 3.2 und damit des Schlittens 3 um eine zu der Ausbringungsrichtung Z_{A} parallele Längsachse verhindert ist. Die Form und die Dimensionierung des Verbindungsstücks 3.2 stellt vielmehr sicher, dass der Schlitten 3 in der Aufnahme 100 ausschließlich entlang der Ausbringungsrichtung Z_{A} - oder mit anderen Worten um eine zu der Ausbringungsrichtung Z_{A} parallele Längsachse - verschieblich ist.

Zwischen den beiden Wangen oder Flanken des Verbindungsstückes 3.2 und den jeweils an einer Flanke oder Wange ausgebildeten Stecköffnungen 3.23 oder 3.24 ist ferner noch eine weitere (dritte) Stecköffnung 3.22 für das Verbindungsstück 3.1 vorgesehen. Diese dritte Stecköffnung 3.22 weist im Querschnitt die Form eines Langlochs auf und erstreckt sich parallel zu den (ersten und zweiten) Stecköffnungen 3.23 und 3.24 innerhalb des Verbindungsstücks 3.2.

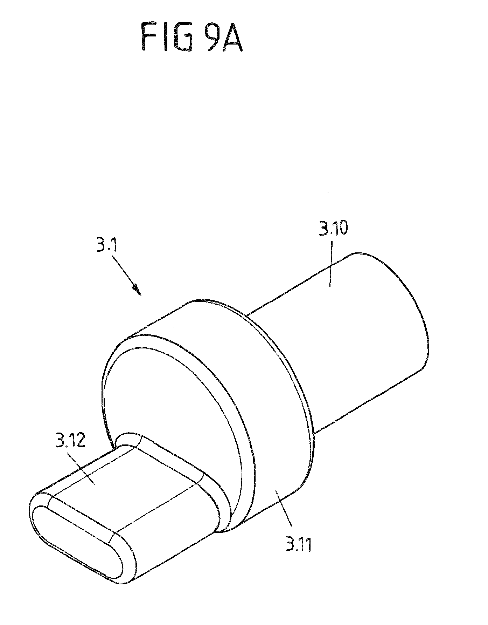

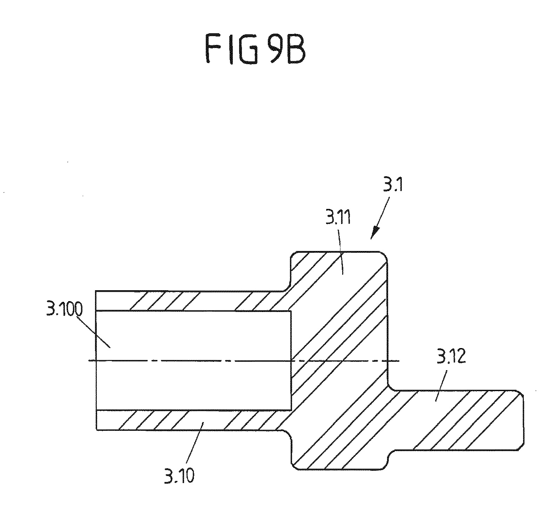

Die dritte mittige Stecköffnung 3.2, die unterhalb der Aussparung 3.21 in dem Verbindungsstück 3.2 ausgebildet ist, so dass die beiden als Durchgangsbohrungen ausgebildeten Stecköffnungen 3.23 und 3.24 symmetrisch hierzu sind, dient der Aufnahme eines Steckzapfens 3.12 des Steckverbinders 3.1 (vergleiche Figuren 9A und 9B).

Der Steckzapfen 3.12 weist eine mit der mittigen Aussparung 3.22 des Verbindungsstücks 3.2 korrespondierende Form auf, so dass er in die Aussparung 3.22 eingesteckt werden kann, um das Verbindungsstück 3.2 und den Steckverbinder 3.1 dauerhaft (kraftschlüssig) miteinander zu verbinden. Der Steckzapfen 3.12 steht dabei von einer ebenen Seite einer scheibenartigen Basis 3.11 des Steckverbinders 3.1 hervor. Auf der dem Steckzapfen 3.12 abgewandten, zweiten Seite der Basis 3.11 steht wiederum ein hülsenartiger Fortsatz 3.10 hervor, in den ein Ende der Schubstange 20 eingesteckt werden kann.

Die scheibenartige Basis 3.11 fungiert im zusammengebauten des Schlittens 3 und Lagerung des Schlittens 3 innerhalb der Aufnahme 100 des Lagerteils 1 auch als Anschlag für den Schlitten 3. Wie zum Beispiel aus den Schnittdarstellungen 4C und 4D ersichtlich ist, liegt die Basis 3.11 an einem im Inneren des Verbindungsbereichs 101 (zwischen Aufnahmeabschnitt 10 und Lagerungsabschnitt 11) des Lagerteils 1 ausgebildeten Absatz 102 an, wenn sich der Schlitten 3 in vollständig eingefahrenen Zustand, also in seiner Ruhelage, innerhalb der Aufnahme 100 befindet. Durch Anlage der Basis 3.11 an dem Absatz 102 ist die Ruhelage des Schlittens 3 genau definiert und eine Verstellung des Schlittens 3 entgegen der Ausbringungsrichtung Z_{A} blockiert. Im Zusammenspiel mit an der Implantationshilfe I angebrachter oder aktiven (d.h., im Arretierzustand befindlichen) Sperre S ist somit der Schlitten 3 in seiner Ruhelage bzw. in seinem eingefahrenen Zustand gehalten und ist weder in die noch entgegen der Ausbringungsrichtung Z_{A} verstellbar.

Über Teile des Schlittens 3 (hier das Verbindungsstück 3.2 und den Steckverbinder 3.1) ist somit sichergestellt, dass sich der Schlitten 3 stufenlos und geradlinig entlang einer Längsachse zwischen einem vollständig eingefahrenen und einem ausgefahrenen Zustand verstellen lässt. Hierbei wäre es selbstverständlich möglich, von der Scheibenform der Basis 3.11 und der korrespondierenden Form des Absatzes 102 abzuweichen und beispielsweise ein T-Förmiges Profil zu verwenden, um eine Ruhelage des Schlittens 3 vorzugeben.

Ferner ist die Länge der Schubstange 20 hier so gewählt, dass die Länge l₂ des an der dem Körper abgewandten Seite aus dem Lagerteil 1 ragenden Schubstangenteils einen maximalen Verstellweg vorgibt, bei dem lediglich die beiden stabförmigen Führungsmittel 3.3, 3.4 (vollständig) aus der Aufnahme 100 herausfahrbar sind, jedoch das die Zwangsführung des Schlittens 3 sicherstellende und gleichzeitig als Verdrehsicherung wirkende Verbindungsstück 3.2 stets innerhalb der Aufnahme 100 verbleibt (vergleiche hierfür auch Figur 4B). Hierdurch wird auch gewährleistet, dass der Schlitten 3 unter keinen Umständen während eines operativen Eingriffs innerhalb des Körpers eines Patienten verbleibt, sondern stets zusammen mit der Implantationshilfe I aus dem Körper entfernt werden kann.

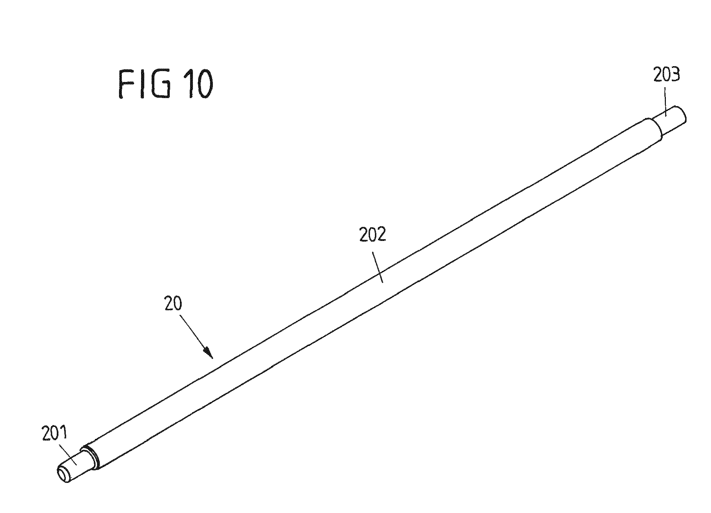

Die Figur 10 zeigt in Einzeldarstellungen noch die gesamte Schubstange 20, die aus einem Mittelteil 202 und zwei jeweils ein Ende der Schubstange 20 definierenden Zapfen 201, 203 ist. Der eine Zapfen 201 ist dabei in eine Bohrung 210 des Griffelements 21 einsteckbar. Der andere Zapfen 203 wiederum ist in eine damit korrespondierende Höhlung 3.100 des hülsenartigen Fortsatzes 3.10 des Steckverbinders 3.1 einsteckbar.

In dem vorliegenden Ausführungsbeispiel ist somit nicht nur der Schlitten 3, sondern die gesamte Implantationshilfe I ausschließlich aus zusammensteckbaren Teilen aufgebaut, so dass die Fertigstellung sowohl kostengünstig als auch schnell und einfach ist.

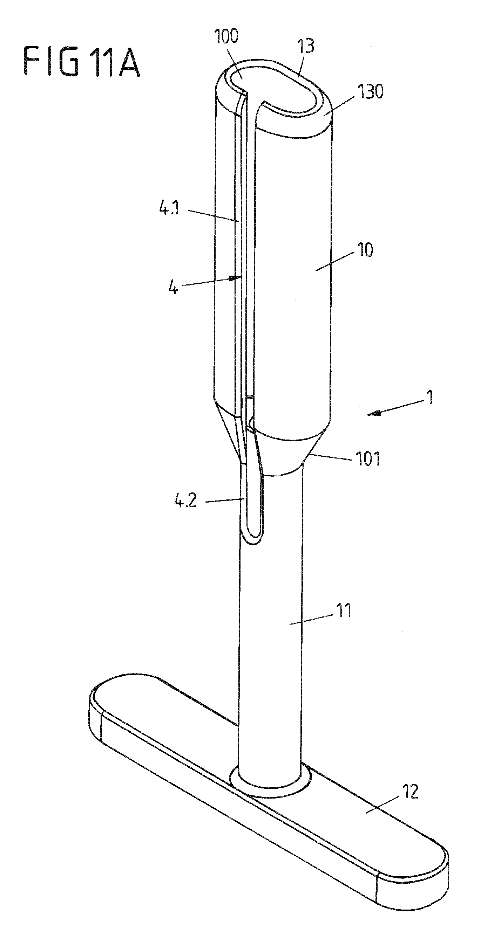

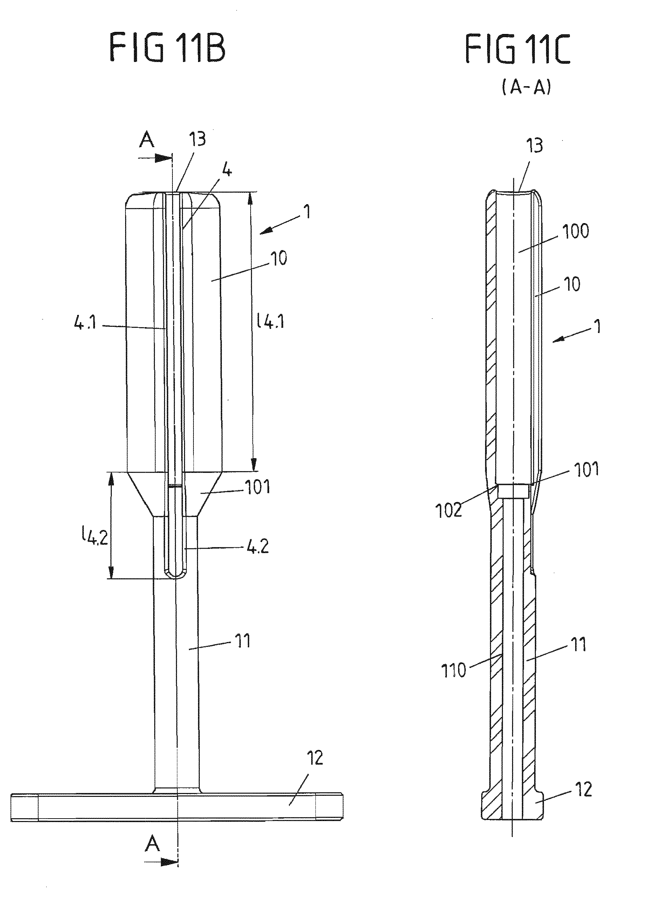

In den Figuren 11A bis 11C ist nochmals im Detail das einstückig ausgeformte Lagerteil 1 in verschiedenen Ansichten veranschaulicht. Dabei zeigt die Figur 11A eine perspektivische Ansicht, die Figur 11B eine Seitenansicht und die Figur 11C eine Schnittansicht des Lagerteils 1 entlang der Figur 11B wiedergegebenen Schnittlinie A-A.

In diesen Detaildarstellungen ist vor allem auch ein sich im Bereich der Aussparung 13 des Lagerteils 100 verjüngender Endabschnitt 130 des Lagerteils 1 besser ersichtlich. Da dieser Endabschnitt 130 den Bereich der Implantationshilfe I bzw. der aus Implantationshilfe I und daran angebrachtem Reservoir R gebildeten Implantationseinheit bildet, der zur Implantierung des Reservoirs R während eines operativen Eingriffs (zuerst) in den Körper des Patienten einzuführen ist, sollte er möglichst leicht und ohne Beschädigung des umliegenden Gewebes eingeführt werden können. Dies wird über die abgerundete sich verjüngende Form des Endabschnitts 130 erreicht. Dieser berandet die Ausbringungsöffnung 13, aus der das Reservoir R ausgebracht wird.

Weiterhin zeigt die Figur 12 in perspektivischer Ansicht die Sperre S in Einzeldarstellung. In dieser Darstellung ist auch die bereits erwähnte Längsnut N gut ersichtlich, die sich über die gesamte Länge der Sperre S erstreckt. Wie bereits zuvor erläutert, erleichtert die Längsnut N ein Aufspreizen der Sperre S, um diese leichter auf den aus dem Lagerteil 1 vorstehenden Abschnitt der Schubstange 20 aufsetzen bzw. von diesem Abschnitt abziehen zu können.

Durch die dargestellte erfindungsgemäße Ausbildung einer Implantationshilfe I bzw. einer entsprechenden Implantationseinheit wird das Einbringen eines (entleerten) Reservoirs R, insbesondere eines Fluid-Reservoirs R einer Penisprothese PP erheblich erleichtert. Gleichzeitig wird sichergestellt, dass ein Reservoir R stets reproduzierbar in einer durch die Implantationshilfe I vorgegebenen Lage an seine bestimmungsgemäße Position innerhalb des Körpers des Patienten gelangt.

So ist nach dem Entfernen einer Sperre S die Schubstange 20 durch den Operateur relativ zu dem Lagerteil 1 der Implantationshilfe I in Ausbringungsrichtung Z_{A} verstellbar. Diese Verstellung wird mittels der Schubstange 20 auf den Schlitten 3 und seine Führungsmittel 3.3 und 3.4 übertragen, die ein starres Gerüst für das Reservoir R vorgeben, das in einem (im Wesentlichen) entleerten und damit in einem noch realtiv schlaffen Zustand vorliegt. Die Länge der Schubstange 20 ist so bemessen, dass der aus dem Lagerteil 1 hervorstehende Teil der Schubstange 20 genauso lang ist wie ein zugelassener Verstellweg, den der Schlitten 3 in der Aufnahme 100 der Implantationshilfe I zurücklegen muss, um das Reservoir R auszubringen. Hat der Operateur die Schubstange 20 vollständig in das Lagerteil 1 eingeschoben, sind die Führungsmittel 3.3, 3.4 des Schlittens 3 und das daran abgebrachte flexible oder sogar elastische Reservoir R aus der Aufnahme 100 herausgetreten.

Ist das Reservoir R elastisch ausgebildet oder ist zumindest der um die Führungsmittel 3.3, 3.4 jeweils herumgeformte Abschnitt einer Hülle des Reservoirs R elastisch, so ist das Reservoir R bestrebt, seine ursprüngliche Form wieder anzunehmen. Es löst sich somit selbsttätig von dem Schlitten 3 und seinen Führungsmitteln 3.3, 3.4, wenn sich der Schlitten 3 in seinem ausgefahrenem Zustand befindet und die Wandungen der Aufnahme 100 eine Rückkehr des Reservoirs R zu seiner ursprünglichen Form nicht mehr verhindern, also die Wandungen der Aufnahme 100 die aufgezwungene Lage des Reservoirs R nicht mehr aufrechterhalten. Im vorliegenden Fall wird sich also das Reservoir R ohne die begrenzende Wirkung der Wandungen der Aufnahme 100 von den stabförmigen Führungsmitteln 3.3, 3.4 des Schlittens 3 entfalten oder abwickeln. Damit verlieren der Schlitten 3 und das Reservoir R den Kontakt miteinander und der Schlitten 3 lässt das Reservoir R im Körper des Patienten zurück.

Nun zieht der Operateur die Schubstange 20 mittels des an ihr befestigten Griffelements 21 wieder in die Ausgangsstellung zurück, die durch Anschlagen des Schlittens 3 bzw. seines Steckverbinders 3.1 an dem Absatz 102 innerhalb des Lagerteils 1 vorgegeben ist. Im Anschluss daran wird die Implantationshilfe I (ohne Reservoir R) aus dem Körper des Patienten entfernt.

Alternativ ist es gleichfalls denkbar, dass das Reservoir R wenigstens abschnittsweise flexibel ausgebildet ist, so dass es sich gleichfalls durch Verformen, zum Beispiel durch Falten oder Wickeln um die Führungselemente 3.3 und 3.4, ohne zusätzliche Haltemittel festlegen lässt. Um nun ein Lösen des Reservoirs R von dem ausgefahrenen Schlitten 3 zu erreichen, ohne dass ein Operateur während eines Eingriffs an dem Reservoir R angreifen muss, kann dann vorgesehen sein, das Reservoir R über seine Leitung L1 mit einem Fluid, zum Beispiel einer Kochsalzlösung, zu befüllen. Durch diese Befüllung spannt sich das flexible Reservoir R bzw. dessen flexible Hülle und das Reservoir R ist hierdurch bestrebt, seine ursprüngliche Form wieder anzunehmen. Die zur Anbringung des Reservoirs R an dem Schlitten 3 und zur Unterbringung in der Aufnahme 100 vorgenommene Verformung des Reservoirs R bildet sich somit durch die Befüllung mit Fluid wieder zurück, so dass die (formschlüssige) Verbindung des Reservoirs R mit dem Schlitten 3 gelöst und das Reservoir R von der Implantationshilfe I freigegeben wird.

### Bezugszeichenliste

- 1: Lagerteil
- 10: Aufnahmeabschnitt
- 100: Aufnahme
- 101: Verbindungsbereich
- 102: Absatz
- 11: Lagerungsabschnitt
- 110: Kanal
- 12: Zwischenstück
- 13: Ausbringungsöffnung
- 130: Endbereich
- 2: Griffteil
- 20: Schubstange
- 201, 203: Zapfen
- 202: Mittelteil
- 21: Griffelement
- 210: Bohrung
- 3: Schlitten
- 3.1: Steckverbinder
- 3.10: Fortsatz
- 3.100: Höhlung
- 3.11: Basis
- 3.12: Steckzapfen
- 3.2: Verbindungsstück
- 3.20: Grundfläche
- 3.21: Aussparung
- 3.22: Stecköffnung
- 3.23,3.24: Stecköffnung
- 3.3,3.4: Führungsmittel
- 3.34: Endabschnitt
- 3.43: Absatz
- 4: Führungskanal
- 4.1,4.2: Führungsabschnitt
- A: Adapterstück
- C: Corpus cavernosum penis
- D: Pumpeinheit
- d: Abstand
- E: Glans penis
- I: Implantationshilfe
- L1, L2, L3: Leitung
- l₂, l₂*: Länge
- l_{4.1}, l_{4.2}: Länge
- N: Längsnut
- P: Penis
- PP: Penisprothese
- R: Reservoir
- R1,R2: Querrand
- R3, R4: Längsrand
- S: Sperrmittel
- SK: Skrotum
- Z: Zylinder
- Z_{A}: Ausbringungsrichtung

## Patentansprüche

1. Implantationshilfe für ein in einen menschlichen oder tierischen Körper implantierbares, mit Fluid befüllbares Reservoir einer Penisprothese, **dadurch gekennzeichnet, dass** die Implantationshilfe (I) ein Lagerteil (1) aufweist, an dem das Reservoir (R) vor seiner Implantierung festlegbar ist und das einen relativ zu dem Lagerteil (1) entlang einer Ausbringungsrichtung (Z_{A}) verstellbaren Schlitten (3) aufweist, wobei mittels des Schlittens (3) das zu implantierende Reservoir (R) in die Ausbringungsrichtung (Z_{A}) verschoben werden kann.

2. Implantationshilfe nach Anspruch 1, **dadurch gekennzeichnet, dass** der Schlitten (3) derart ausgebildet ist, dass das Reservoir (R) an dem Schlitten (3) anbringbar ist.

3. Implantationshilfe nach Anspruch 1 oder 2, **dadurch gekennzeichnet, dass** der Schlitten (3) wenigstens ein stabförmiges Führungsmittel (3.3; 3.4) aufweist, mittels dem das Reservoir (R) in einer vorgegebenen Lage entlang der Ausbringungsrichtung (Z_{A}) verschiebbar ist und/oder an dem das Reservoir (R) befestigbar ist.

4. Implantationshilfe nach einem der Ansprüche 1 bis 3, **dadurch gekennzeichnet, dass** das Lagerteil (1) eine Aufnahme (100) aufweist, die dazu vorgesehen und eingerichtet ist, das zu implantierende Reservoir (R) vor seiner Implantierung zumindest teilweise in seinem Inneren aufzunehmen, und aus der das Reservoir (R) mittels des Schlittens (3) herausschiebbar ist.

5. Implantationshilfe nach Ansprüchen 3 und 4, **dadurch gekennzeichnet, dass** das Führungsmittel (3.3; 3.4) aus der Aufnahme (100) heraus verschiebbar ist.

6. Implantationshilfe nach einem der vorhergehenden Ansprüche, **dadurch gekennzeichnet, dass** das Lagerteil (1) eine Führung (4) aufweist und die Führung (4) eine Führungsbahn vorgibt, entlang der ein Teil (L1) des zu implantierenden Reservoirs (R) während einer durch den Schlitten (3) verursachten Verschiebung des Reservoirs (R) gleitet.

7. Implantationshilfe nach einem der Ansprüche 3 oder 4 und dem Anspruch 5, **dadurch gekennzeichnet, dass** die Führung (4) an der Aufnahme (100) vorgesehen ist und/oder die Führung (4) dazu vorgesehen und eingerichtet ist, eine Führungsbahn für ein nicht im Inneren der Aufnahme (100) aufzunehmendes Teil (L1) des Reservoirs (R) vorzugeben.

8. Implantationshilfe nach einem der vorhergehenden Ansprüche, **dadurch gekennzeichnet, dass** die Implantationshilfe (I) zur manuellen Bedienbarkeit vorgesehen und eingerichtet ist und/oder eine Sperre (S) aufweist, die in einem Arretierzustand an der Implantationshilfe festlegbar ist, in dem die Sperre (S) zumindest eine Verstellung des Schlittens (3) in die Ausbringungsrichtung (Z_{A}) verhindert.

9. Implantationshilfe nach einem der vorhergehenden Ansprüche, **dadurch gekennzeichnet, dass** der Schlitten (3) und/oder die Implantationshilfe (I) ausschließlich aus zusammengesteckten Bauteilen (3.1, 3.2, 3.3, 3.4; 1, 20, 21, 3.1, 3.2, 3.3, 3.4) gebildet ist.

10. Implantationshilfe nach einem der vorhergehenden Ansprüche, **dadurch gekennzeichnet, dass** die Implantationshilfe (I) dazu vorgesehen und eingerichtet ist, dass ein Reservoir (R) mit einer zumindest teilweise flexiblen Hülle daran festlegbar ist.

11. Implantationseinheit mit einer Implantationshilfe (I) nach einem der Ansprüche 1 bis 10 und mit einem in einen menschlichen oder tierischen Körper implantierbaren, mit Fluid befüllbaren Reservoir (R) einer Penisprothese, das an dem Lagerteil (1) der Implantationshilfe (I) lösbar festlegt ist und zur Implantierung mittels des Schlittens (3) relativ zu dem Lagerteil (1) in die Ausbringungsrichtung (Z_{A}) verschiebbar ist.

12. Verfahren zur Herstellung einer Implantationseinheit mit implantierbarem und mit Fluid befüllbarem Reservoir einer Penisprothese, insbesondere einer Implantationseinheit nach Anspruch 11, mit den folgenden Schritten:
- Bereitstellung einer Implantationshilfe (I) mit einem Lagerteil (1), an dem ein zu implantierendes Reservoir (R) festlegbar ist und das einen relativ zu dem Lagerteil (1) entlang einer Ausbringungsrichtung (Z_{A}) verstellbaren Schlitten (3) aufweist;
- Verstellung des Schlittens (3) in Ausbringungsrichtung (Z_{A});
- Anbringung des Reservoirs (R) an dem Schlitten (3;
- Verstellung des Schlittens (3) mit daran angebrachtem Reservoir (R) entgegen der Ausbringungsrichtung (Z_{A}).

13. Verfahren nach Anspruch 12, **dadurch gekennzeichnet, dass** das Reservoir (R) eine zumindest teilweise flexible Hülle aufweist und wenigstens ein flexibler Abschnitt (R3; R4) der Hülle zur Festlegung des Reservoirs (R) an dem Schlitten (3) verformt, insbesondere gewickelt oder gefaltet wird.

## Claims

1. Implantation aid for a reservoir of a penile prosthesis, which reservoir can be implanted in a human or animal body and can be filled with fluid, **characterized in that** the implantation aid (I) has a bearing part (1), on which the reservoir (R) can be secured before being implanted and which has a carriage (3) that is adjustable relative to the bearing part (1) along an output direction (Z_{A}), wherein the reservoir (R) to be implanted can be moved in the output direction (Z_{A}) by means of the carriage (3).

2. Implantation aid according to Claim 1, **characterized in that** the carriage (3) is designed in such a way that the reservoir (R) can be mounted on the carriage (3).

3. Implantation aid according to Claim 1 or 2, **characterized in that** the carriage (3) has at least one rod-shaped guide means (3.3; 3.4), by means of which the reservoir (R) can be moved in a predetermined position along the output direction (Z_{A}) and/or on which the reservoir (R) can be secured.

4. Implantation aid according to one of Claims 1 to 3, **characterized in that** the bearing part (1) has a recess (100), which is provided and configured for the purpose of at least partially receiving, in its interior, the implantable reservoir (R) before the latter is implanted, and from which the reservoir (R) can be pushed out by means of the carriage (3).

5. Implantation aid according to Claims 3 and 4, **characterized in that** the guide means (3.3; 3.4) is movable out of the recess (100).

6. Implantation aid according to one of the preceding claims, **characterized in that** the bearing part (1) has a guide (4), and the guide (4) predefines a guide path along which a part (L1) of the reservoir (R) to be implanted slides during a movement of the reservoir (R) caused by the carriage (3).

7. Implantation aid according to either of Claims 3 and 4 and according to Claim 5, **characterized in that** the guide (4) is provided on the recess (100) and/or the guide (4) is provided and configured for the purpose of predefining a guide path for a part (L1) of the reservoir (R) that is not to be received in the interior of the recess (100).

8. Implantation aid according to one of the preceding claims, **characterized in that** the implantation aid (I) is provided and configured for manual operation and/or has a blocking means (S) that can be secured in a locking state on the implantation aid, by the blocking means (S) preventing at least an adjustment of the carriage (3) in the output direction (Z_{A}).

9. Implantation aid according to one of the preceding claims, **characterized in that** the carriage (3) and/or the implantation aid (I) is formed exclusively from interconnected structural parts (3.1, 3.2, 3.3, 3.4; 1, 20, 21, 3.1, 3.2, 3.3, 3.4).

10. Implantation aid according to one of the preceding claims, **characterized in that** the implantation aid (I) is provided and configured such that a reservoir (R) can be secured thereon with an at least partially flexible sleeve.

11. Implantation unit with an implantation aid (I) according to one of Claims 1 to 10 and with a reservoir (R) of a penile prosthesis, which reservoir (R) can be implanted in a human or animal body and can be filled with fluid, and which reservoir (R) is secured releasably on the bearing part (1) of the implantation aid (I) and, for implantation, is movable relative to the bearing part (1) in the output direction (Z_{A}) by means of the carriage (3).

12. Method for producing an implantation unit with an implantable reservoir of a penile prosthesis that can be filled with fluid, in particular an implantation unit according to Claim 11, said method having the following steps:
- making available an implantation aid (I) with a bearing part (1) on which a reservoir (R) to be implanted can be secured and which has a carriage (3) adjustable relative to the bearing part (1) in an output direction (Z_{A});
- adjusting the carriage (3) in output direction (A) ;
- mounting the reservoir (R) on the carriage (3);
- adjusting the carriage (3), with the reservoir (R) mounted thereon, counter to the output direction (Z_{A}).

13. Method according to Claim 12, **characterized in that** the reservoir (R) has an at least partially flexible sleeve, and at least a flexible portion (R3; R4) of the sleeve is deformed, in particular wound or folded, in order to secure the reservoir (R) on the carriage (3).

## Revendications

1. Auxiliaire d'implantation pour un réservoir d'une prothèse pénienne implantable dans un corps humain ou animal, pouvant être rempli de fluide, **caractérisé en ce que** l'auxiliaire d'implantation (I) présente une partie de support (1) sur laquelle peut être fixé le réservoir (R) avant son implantation et qui présente un chariot (3) déplaçable le long d'une direction de sortie (Z_{A}) par rapport à la partie de support (1), le réservoir à implanter (R) pouvant être déplacé dans la direction de sortie (Z_{A}) au moyen du chariot (3).

2. Auxiliaire d'implantation selon la revendication 1, **caractérisé en ce que** le chariot (3) est réalisé de telle sorte que le réservoir (R) puisse être monté sur le chariot (3).

3. Auxiliaire d'implantation selon la revendication 1 ou 2, **caractérisé en ce que** le chariot (3) présente au moins un moyen de guidage en forme de barre (3.3 ; 3.4) au moyen duquel le réservoir (R) peut être déplacé dans une position prédéfinie le long de la direction de sortie (Z_{A}) et/ou au niveau duquel le réservoir (R) peut être fixé.

4. Auxiliaire d'implantation selon l'une quelconque des revendications 1 à 3, **caractérisé en ce que** la partie de support (1) présente un logement (100) qui est prévu et adapté pour recevoir au moins en partie en son intérieur le réservoir à implanter (R) avant son implantation, et hors duquel le réservoir (R) peut être ressorti au moyen du chariot (3).

5. Auxiliaire d'implantation selon les revendications 3 et 4, **caractérisé en ce que** le moyen de guidage (3.3 ; 3.4) peut être déplacé hors du logement (100).

6. Auxiliaire d'implantation selon l'une quelconque des revendications précédentes, **caractérisé en ce que** la partie de support (1) présente un guide (4) et le guide (4) prédéfinit une trajectoire de guidage le long de laquelle une partie (L1) du réservoir à implanter (R) glisse pendant un déplacement du réservoir (R) provoqué par le chariot (3).

7. Auxiliaire d'implantation selon l'une quelconque des revendications 3 ou 4, et selon la revendication 5, **caractérisé en ce que** le guide (4) est prévu au niveau du logement (100) et/ou le guide (4) est prévu et adapté pour prédéfinir une trajectoire de guidage pour une partie (L1) du réservoir (R) ne devant pas être reçue à l'intérieur du logement (100).

8. Auxiliaire d'implantation selon l'une quelconque des revendications précédentes, **caractérisé en ce que** l'auxiliaire d'implantation (I) est prévu et adapté pour être actionné manuellement et/ou présente un verrouillage (S) qui peut être fixé dans un état d'arrêt au niveau de l'auxiliaire d'implantation, dans lequel le verrouillage (S) empêche au moins un déplacement du chariot (3) dans la direction de sortie (Z_{A}).

9. Auxiliaire d'implantation selon l'une quelconque des revendications précédentes, **caractérisé en ce que** le chariot (3) et/ou l'auxiliaire d'implantation (I) sont formés exclusivement de composants enfichés les uns dans les autres (3.1, 3.2, 3.3, 3.4 ; 1, 20, 21, 3.1, 3.2, 3.3, 3.4).

10. Auxiliaire d'implantation selon l'une quelconque des revendications précédentes, **caractérisé en ce que** l'auxiliaire d'implantation (I) est prévu et adapté de manière à ce qu'un réservoir (R) avec une enveloppe au moins partiellement flexible puisse être fixée sur celui-ci.

11. Unité d'implantation comprenant un auxiliaire d'implantation (I) selon l'une quelconque des revendications 1 à 10 et comprenant un réservoir (R) d'une prothèse pénienne implantable dans un corps humain ou animal, pouvant être rempli de fluide, qui est fixé de manière amovible sur la partie de support (1) de l'auxiliaire d'implantation (I) et qui peut être déplacé pour l'implantation au moyen du chariot (3) par rapport à la partie de support (1) dans la direction de sortie (Z_{A}).

12. Procédé de fabrication d'une unité d'implantation comprenant un réservoir d'une prothèse pénienne implantable et pouvant être rempli de fluide, en particulier d'une unité d'implantation selon la revendication 11, comprenant les étapes suivantes
- fourniture d'un auxiliaire d'implantation (I) avec une partie de support (1) sur laquelle peut être fixé un réservoir à implanter (R) et qui présente un chariot (3) déplaçable par rapport à la partie de support (1) le long d'une direction de sortie (Z_{A}) ;
- déplacement du chariot (3) dans la direction de sortie (Z_{A}) ;
- montage du réservoir (R) sur le chariot (3) ;
- déplacement du chariot (3) avec le réservoir (R) monté sur celui-ci dans le sens inverse de la direction de sortie (Z_{A}).

13. Procédé selon la revendication 12, **caractérisé en ce que** le réservoir (R) présente une enveloppe au moins partiellement flexible et au moins une portion flexible (R3 ; R4) de l'enveloppe est déformée, notamment enroulée ou pliée, pour fixer le réservoir (R) sur le chariot (3).
